# EUROPÄISCHE PATENTSCHRIFT

(11) **EP 3 143 407 B1**
(45) Veröffentlichungstag und Bekanntmachung des Hinweises auf die Patenterteilung: **07.11.2018**
(21) Anmeldenummer: 14753004.2
(22) Anmeldetag: 10.05.2014
(51) Int. Cl.: G01N 33/74, G01N 1/28

(54) **VERFAHREN ZUR BESTIMMUNG VON VITELLOGENIN ALS BIOMARKER FÜR EINE EXOGENE ÖSTROGENE WIRKUNG AUF FISCHE**
METHOD FOR DETERMINATION OF VITELLOGENIN AS BIOMARKER FOR AN EXOGENOUS OESTROGENIC EFFECT ON FISH
PROCÉDÉ POUR DÉTERMINER LA PRÉSENCE DE LA VITELLOGÉNINE COMME BIOMARQUEUR D'UNE ACTIVITÉ STROGÉNIQUE EXOGÈNE SUR LES POISSONS

(43) Veröffentlichungstag der Anmeldung: 22.03.2017
(73) Patentinhaber: Gobio GmbH, 65326 Aarbergen/Kettenbach (DE); Teco Medical AG, 4450 Sissach (CH)
(72) Erfinder: ALLNER, Bernhard, 65326 Aarbergen / Kettenbach (DE); HENNIES, Mark, CH-4450 Sissach (CH)
(74) Vertreter: Mackert, Andreas
(86) Internationale Anmeldenummer: PCT/DE2014/100161
(87) Internationale Veröffentlichungsnummer: WO 2015/172756

(56) Entgegenhaltungen:
- AUGUSTINE ARUKWE ET AL: "Molecular and cellular detection of expression of vitellogenin and zona radiata protein in liver and skin of juvenile salmon (Salmo salar) exposed to nonylphenol", CELL AND TISSUE RESEARCH, SPRINGER, BERLIN, DE, Bd. 331, Nr. 3, 21. Dezember 2007 (2007-12-21), Seiten 701-712, XP019590717, ISSN: 1432-0878
- REY VAZQUEZ G ET AL: "Exposure to waterborne 4-tert-octylphenol induces vitellogenin synthesis and disrupts testis morphology in the South American freshwater fish Cichlasoma dimerus (Teleostei, Perciformes)", COMPARATIVE BIOCHEMISTRY AND PHYSIOLOGY PART C: TOXICOLOGY & PHARMACOLOGY, ELSEVIER, US, Bd. 150, Nr. 2, 1. August 2009 (2009-08-01), Seiten 298-306, XP026266049, ISSN: 1532-0456, DOI: 10.1016/J.CBPC.2009.05.012 [gefunden am 2009-05-27]
- DENSLOW NANCY D ET AL: "Vitellogenin as a biomarker of exposure for estrogen or estrogen mimics", ECOTOXICOLOGY, CHAPMAN & HALL, LONDON, GB, Bd. 8, Nr. 5, 1. Oktober 1999 (1999-10-01) , Seiten 385-398, XP002399650, ISSN: 0963-9292, DOI: 10.1023/A:1008986522208

## Beschreibung

Es ist notwendig, die Wirkung von in der Umwelt vorhandenen Chemikalien auf Lebewesen, insbesondere auf Wirbeltiere, regelmäßig zu untersuchen. Dabei kann unter anderem untersucht werden, ob Chemikalien und/oder deren Abbauprodukte beispielsweise eine Wirkung auf das endokrine System haben.

Zahlreiche Untersuchungen haben in diesem Zusammenhang bereits gezeigt, dass insbesondere Östrogeneffekte durch eine Interaktion von Chemikalien mit den Östrogenrezeptoren bei Wirbeltieren, im Speziellen bei Fischen, vorgetäuscht werden, so dass es z.B. zu Reproduktionsstörungen bei männlichen Fischen nach Exposition mit Abwässern kommt. Im Rahmen der Chemikalientestung, die nach genauen Vorgaben von OECD Testguidelines erfolgt, wird zur Zeit nach gängigen Testverfahren eine immunologische Erfassung des Vitellogenins mittels Antikörpern durchgeführt, wobei aus Ganzkörperhomogenaten oder im Blutserum/-plasma von Testfischen die paradoxe Induktion von Dotterproteinen (Vitellogenin) bestimmt wird. Dieses Verfahren wird z.B. beschrieben in dem Abschlussbericht BMBF-Förderkennzeichen 02WA9981/9 des Hessischen Landesamtes für Umwelt und Geologie, Februar 2003.

Bei eierlegenden Tieren ist Vitellogenin ein Eidotter-Vorläuferprotein, welches durch östrogene Induktion während der Hauptphase des Oozytenwachstums in der Leber gebildet wird. Dieses Dotterprotein wandert im Blutstrom der weiblichen Fische zu den Ovarien, zerfällt dort in andere Dotterproteine, die später als Nahrungsvorrat für die sich entwickelnden Fischembryos im Ei dienen. Kommt es nun durch Xenoöstrogene in Gewässern zu einer künstlich induzierten Vitellogenin-Synthese in männlichen und jungen Fischen, z.B. durch Verunreinigung des Gewässers mit Abwässern, dann sind Reproduktionsstörungen bei diesen Fischen die Folge.

Mit Hilfe der bislang verwandten Vitellogenin-Tests kann bei Fischen der Östrogenstatus bestimmt werden, der Status der sexuellen Entwicklung, die Identifizierung der Geschlechter, wenn kein Geschlechtsdimorphismus vorhanden ist, es kann der Nachweis von östrogener Wirkung bei natürlichen oder anthropogenen Substanzen oder Chemikalien geführt werden sowie eine Untersuchung von Gewässern oder Abwässern auf östrogenwirksame Substanzen erfolgen.

Die bislang bekannten Testverfahren sind jedoch für die in der Chemikalientestung oder im Umweltmonitoring gebräuchlichen Testfische destruktiv, d.h. sie erfordern insbesondere im Umweltmonitoring die Tötung von Fischen. Außerdem erfordern die konventionellen Tests zur Chemikalientestung immer eine gesonderte Durchführung und erfordern damit einen zusätzlichen Tierverbrauch.

Zudem kann die gewünschte paradoxe Induktion von Vitellogenin nach längerer Exposition über Rückkopplungsmechanismen gehemmt werden, so dass die konventionellen Tests wenig sensitiv sind.

Nachteilig ist ebenfalls bei den bisher verwendeten Testverfahren, dass die Matrix, aus der die Bestimmung erfolgt, nur schwer zu definieren ist und großen Schwankungen unterliegt. Gerade bei der Entnahme von Blutproben kommt es häufig zu Kontaminationen durch Gewebsflüssigkeit.

Auch sind die Probenvorbereitung wie die Serumgewinnung oder Herstellung geeigneter Homogenate bislang sehr aufwändig und kaum standardisierbar.

Vitellogenin ist ein sehr instabiles Protein. Sowohl das Protein als Zielanalyt in seiner biologischen Matrix (in der Probe), als auch Standardlösungen mit definierten Proteingehalten zerfallen. Konventionelle Tests eignen sich daher nicht für eine absolute Quantifizierung, da auch Bruchstücke immunologisch erfasst werden.

Schließlich sind die bekannten konventionellen Tests in der Regel nur für eine geringe Zahl sehr nahe verwandter Spezies anwendbar.

In dem Artikel Augustine Arukwe et al: "Molecular and cellular detection of expression of vitellogenin and zona radiata protein in liver and skin of juvenile salmon (Salmo salr) exposed to nonylphenol", Cell an dtissue research, Springer, Berlin, DE, Bd. 331, Nr. 3, 21.12.2007 (2007-12-21), Seiten 701-712, XP019590717, ISSN: 1432-0878, wird ein Verfahren beschrieben, in welchem Vitellogenin im Hautschleim von Lachsen als Biomarker detektiert wird, wobei größere Mengen Hautschleims als Proben mittels eines Plastikspatels abgenommen werden, wofür die Versuchstiere betäubt werden müssen. Eine solche Betäubung der Fische bedeutet einen massiven Eingriff in den experimentellen Ablauf und stellt einen großen Nachteil der dort beschriebenen Methode dar. Zudem sind Lachse als Versuchstiere aufgrund ihrer Größe und komplexen Haltungsbedingungen für schnelle und unkomplizierte Standardtests ungeeignet, im beschriebenen Verfahren aber notwendig, da dort größere Mengen Hautschleims für die nachfolgenden Analysen benötigt werden.

Aus dem Artikel Rex Vazquez et al: "Exposure to waterborne 4-tert-octylphenol induces vitellogenin synthesis and disrupts testis morphology in the South American freshwater fish Cichlasoma dimerus (Teleostei, Perciformes)",Comparative Biochemistry and Physiology Part C: Toxicology & Pharmacology, Elsevier, US, Bd. 150, Nr. 2, 01.08.2009. (2009-08-01), Seiten 298-306, XP026266049, ISSN: 1532-0456, ist ebenfalls eine Analysemethode der Vitellogeninbestimmung aus dem Hautschleim von Fischen bekannt, bei welcher wiederum an größeren Fischen von ca. 10 - 15 cm Größe Hautschleim mittels eines Metallspatels abgeschabt wird, die Fische jedoch ebenfalls vor dieser Prozedur betäubt werden müssen, um genügend Hautschleim zu erhalten. Damit ist dieses bekannte Verfahren als einfaches und unkompliziertes Standardverfahren wie es die OECD fordert, nicht geeignet.

Im Artikel "Denslow, Nancy et al: "Vitellogenin as a biomarker of exposure for estrogen or estrogen mimics", Ecotoxicology, Chapman & Hall, London, GB, Bd. 8, Nr. 5,01.10.1999 (199-10-01), Seiten 385-398, XP002399650, ISSN: 0963-9292, wird ebenfalls Vitellogenin als Biomarker verwendet, allerdings aus Plasma von Fischen, nicht aus Hautschleim. Die Fische werden für diese Analysemethode mehrfach betäubt, insbesondere auch bei der Blutentnahme zur Plasmagewinnung, und es werden wiederum große Fischarten benötigt, um genügend große Blutgefäße für die Blutentnahme zu haben.

Vor diesem Hintergrund ist die Aufgabe des offenbarten erfinderische Verfahren zur Bestimmung von Vitellogenin als Biomarker bzw. Endpunkt für eine exogene östrogene Wirkung auf Fische ein nicht-destruktives Verfahren zu schaffen, bei welchem die Versuchstiere nicht getötet, betäubt oder körperlich verletzt werden müssen. Das Verfahren soll hierbei auch keinen zusätzlichen Verbrauch von Versuchstieren zur Durchführung von Tests auf östrogene, antiöstrogene und endokrine Disruptoren erfordern.

Das erfindungsgemäße Verfahren zur Bestimmung von Vitellogenin als Biomarker bzw. Endpunkt für eine exogene östrogene Wirkung auf Fische umfasst die Verfahrensschritte der
a) Abnahme einer Hautschleimprobe auf der dorsalen Körperregion eines Fisches durch Abstreichen mittels eines forensischen Swabs mit einer Sollbruchstelle,
b) Überführung des mit Hautschleim behafteten Teils des Swabs nach Bruch an der Sollbruchstelle in ein passendes Reaktionsgefäß,
c) Zugabe von proteinstabilisierendem Puffer in das Reaktionsgefäß,
d) Homogenisieren der Hautschleimprobe,
d) Entnahme eines Aliquots der Hautschleimprobe zur Vitellogeninbestimmung mittels eines entsprechenden ELISA Nachweisverfahrens.

Es handelt sich demnach bei dem erfindungsgemäßen Verfahren um ein nicht-destruktives Verfahren, bei welchem die Versuchstiere nicht getötet, betäubt oder verletzt werden müssen und auch kein zusätzlicher Verbrauch von Versuchstieren zur Durchführung von Tests auf östrogene, antiöstrogene und endokrine Disruptoren erforderlich ist.

Nach einer Expositionszeit von 4-7 Tagen kann die Wirksamkeit der exogenen Noxe (schädliche Substanzen) zum Zeitpunkt der maximalen Induktion von Vitellogenin und damit der optimalen speziesspezifischen Sensitivität erfasst werden.

Die östrogene Wirksamkeit kann als zusätzlicher Endpunkt, also gekoppelt an subchronische Langzeittests wie OECD 215, oder in subletalen Konzentrationen des Akuttests OECD 203 getestet werden. Es ist hierfür kein zusätzlicher experimenteller Aufwand erforderlich.

Die im offenbarten erfindungsgemäßen Verfahren offenbarte Matrix (Hautschleim) ist im Gegensatz zu konventionellen Tests gut definiert. Die Proben müssen nicht von Nicht-Zielgeweben wie Blut, Lymphe, Epithelien u.a. separiert werden.

Auch sind nach dem erfindungsgemäßen Verfahren keine aufwändigen Probenvorbereitungen notwendig.

Zweckmäßigerweise wird in einer Erweiterung des Verfahrens ein weiteres Aliquot der homogenisierten Hautschleimprobe als Parallelprobe entnommen und eine weitere Vitellogeninbestimmung durch ein ELISA Nachweisverfahren durchgeführt. Auf diese Weise wird der erste Nachweis an dieser Parallelprobe überprüft und eine höhere Sicherheit in Bezug auf die Ergebnisse des Nachweisverfahrens erreicht.

Ein weiteres Aliquot der homogenisierten Hautschleimprobe kann in einer vorteilhaften Verfahrenserweiterung entnommen und eine Gesamtproteinbestimmung durchgeführt werden, da so eine Bestimmung der Relation des Gesamtproteingehaltes zum zuvor bestimmten Vitellogeningehalt der Hautschleimprobe möglich wird, die weitere Aufschlüsse gibt. Diese Gesamtproteinbestimmung kann hierbei sowohl bei einem Verfahren nach dem Hauptanspruch wie auch bei einem Verfahren in Kombination mit der Parallelprobe nach Anspruch 2 erfolgen.

Die Abnahme der Hautschleimprobe des Fisches erfolgt hierbei sinnvollerweise mittels eines forensischen Swabs mit zusätzlicher Sollbruchstelle zur Vermeidung der Kompression der Hautschleimzellen, die bei Probennahme mittels Metall- oder Kunststoffspateln wie im Stand der Technik beschrieben, immer teilweise komprimiert und zerstört werden, auf der dorsalen Körperregion des Fisches. Es hat sich ergeben, dass diese Körperregion des Fisches für die Entnahme der Hautschleimprobe in besonderem Maße geeignet ist.

Die Verwendung des forensischen Swabs mit zusätzlicher Sollbruchstelle hat hierbei insbesondere in Kombination mit einem Verfahrensablauf Vorteile, bei dem der abgebrochene Teil des forensischen Swabs als Rührkörper im Reaktionsgefäß beim Homogenisieren der Hautschleimprobe verbleibt. Hier kann zweckmäßigerweise ein Mikroreaktionsgefäß Verwendung finden, welches geeignet und geformt ist, die Hautschleimprobe mitsamt dem Teilstück des Swabs aufzunehmen. Der nächste Verfahrensschritt ist die Zugabe eines proteinstabiliserenden Puffers in das Reaktionsgefäß. Durch die Verwendung geeigneter Puffer wird dem Zerfall des Proteins vorgebeugt, wodurch der Test für eine größere Anzahl von Spezies (taxonomischen Gruppen) wie z.B. Karpfenartige, anwendbar ist.

Für die Durchführung des Nachweisverfahrens nach der Homogenisierung der Probe hat sich als vorteilhaft erwiesen, das Aliquot zur weiteren Bestimmung auf eine Mikrotiterplatte zu überführen. So ist eine labortechnisch standardisierte Auswertung leicht möglich. Dies gilt insbesondere für das angesprochene ELISA Verfahren als antikörperbasiertes Nachweis- bzw. Immunoassay-Verfahren basierend auf einer enzymatischen Farbreaktion.

Das erfindungsgemäße Verfahren ist bislang getestet und geeignet für mehrere Fischgruppen, insbesondere für karpfenartige Fische, für barschartige Fische und für lachsartige Fische, wobei bei den karpfenartigen Fischen solche der Art Karpfen (Cyprinus carpio), Goldfisch (Carrasius auratus), Plötze (Rutilus rutilus), Zebrafisch (Danio rerio)oder Dickkopfelritze (Pimephales promelas) offenbart werden.

Für die barschartigen Fische wurden vorteilhafterweise Fische der Art Flußbarsch (Perca fluviatilis), Tilapia (Oreochromis niloticus), Stichling (Gasterosteus aculeatus), Reisfisch (Oryzias latipes) oder australische Regenbogenfische (Melanotaenis spec.) eingesetzt und bei den lachsartigen Fischen kamen vorteilhafterweise solche der Art Bachforelle (Salmo trutta forma fario), Regenbogenforelle (Oncorhynchus mykiss oder Saibling (Salvelinus alpinus) als geeignete Versuchstiere zum Einsatz.

Das offenbarte erfindungsgemäße Verfahren kann unter anderem eingesetzt werden für den Nachweis von östrogenen Wirkungen in Laborversuchen als zusätzlicher Endpunkt in Standardverfahren, zur Bestimmung der Laichreife von weiblichen karpfenartigen Fischen und zur Erfassung der Exposition gegenüber östrogen wirksamen Umweltnoxen im Freiland. Auch eine Erfassung einer Störung der Geschlechtsreife durch antiöstrogene Umweltnoxen im Freiland ist mit diesem Verfahren einfach möglich.

## Patentansprüche

1. Verfahren zur Bestimmung von Vitellogenin im Hautschleim von Fischen als Biomarker für eine exogene östrogene Wirkung auf Fische,
**gekennzeichnet durch** die Verfahrensschritte
a) Abnahme einer Hautschleimprobe auf der dorsalen Körperregion eines Fisches durch Abstreichen mittels forensischem Swab mit einer Sollbruchstelle,
b) Überführung des mit Hautschleim behafteten Teils des Swabs nach Bruch an der Sollbruchstelle in ein passendes Reaktionsgefäß,
c) Zugabe von proteinstabilisierendem Puffer in das Reaktionsgefäß,
d) Homogenisieren der Hautschleimprobe,
e) Entnahme eines Aliquots der Hautschleimprobe und Vitellogeninbestimmung mittels eines entsprechenden ELISA Nachweisverfahrens.

2. Verfahren nach Anspruch 1,
**dadurch gekennzeichnet, dass**
ein weiteres Aliquot der homogenisierten Hautschleimprobe als Parallelprobe entnommen und eine weitere Vitellogeninbestimmung durch ein ELISA Nachweisverfahren durchgeführt wird.

3. Verfahren nach Anspruch 1,
**dadurch gekennzeichnet, dass**
ein weiteres Aliquot der homogenisierten Hautschleimprobe entnommen und eine Gesamtproteinbestimmung durchgeführt wird zur Bestimmung der Relation des Gesamtproteingehaltes zum Vitellogeningehalt der Hautschleimprobe.

4. Verfahren nach einem der Ansprüche 1 bis 3,
**dadurch gekennzeichnet, dass**
das Aliquot zur weiteren Bestimmung auf eine Mikrotiterplatte überführt wird.

5. Verfahren nach Anspruch 1,
**dadurch gekennzeichnet, dass**
es sich bei den Fischen um karpfenartige Fische handelt.

6. Verfahren nach Anspruch 1,
**dadurch gekennzeichnet, dass**
es sich bei den Fischen um barschartige Fische handelt.

7. Verfahren nach Anspruch 1,
**dadurch gekennzeichnet, dass**
es sich bei den Fischen um lachsartige Fische handelt.

8. Verfahren nach Anspruch 5,
**dadurch gekennzeichnet, dass**
die karpfenartigen Fische der Art Karpfen (Cyprinus carpio), Goldfisch (Carrasisus auratus), Plötze (Rutilus rutilus), Zebrafisch (Danio rerio)oder Dickkopfelritze (Pimephales promelas) entstammen.

9. Verfahren nach Anspruch 6,
**dadurch gekennzeichnet, dass**
die barschartigen Fische der Art Flußbarsch (Perca fluviatilis), Tilapia (Oreochromis niloticus), Stichling (Gasterosteus aculeatus), Reisfisch (Oryzias latipes) oder australische Regenbogenfische (Melanotaenis spec.) entstammen.

10. Verfahren nach Anspruch 7,
**dadurch gekennzeichnet, dass**
die lachsartigen Fische der Art Bachforelle (Salmo trutta forma fario), Regenbogenforelle (Oncorhynchus mykiss) oder Saibling (Salvelinus alpinus) entstammen.

11. Verfahren nach einem der vorangegangenen Ansprüche,
**dadurch gekennzeichnet, dass**
als Reaktionsgefäß ein Mikroreaktionsgefäß verwendet wird.

## Claims

1. Method for determining vitellogenin in the skin mucus of fishes as biomarker for an exogenous estrogenic effect on fishes,
**characterized by** the method steps of
a) taking a skin mucus sample on the dorsal body region of a fish by swabbing by means of a forensic swab with a predetermined breaking point,
b) transferring the part of the swab laden with skin mucus after breaking at the predetermined breaking point to a suitable reaction vessel,
c) adding protein-stabilizing buffer to the reaction vessel,
d) homogenizing the skin mucus sample,
e) removing an aliquot of the skin mucus sample and vitellogenin determining by means of an appropriate ELISA detection method.

2. Method according to Claim 1,
**characterized in that**
a further aliquot of the homogenized skin mucus sample is removed as parallel sample and a further vitellogenin determination by means of an ELISA detection method is carried out.

3. Method according to Claim 1,
**characterized in that**
a further aliquot of the homogenized skin mucus sample is removed and a total protein determination is carried out in order to determine the relationship of the total protein content to the vitellogenin content of the skin mucus sample.

4. Method according to any of Claims 1 to 3,
**characterized in that**
the aliquot is transferred to a microtiter plate for further determination.

5. Method according to Claim 1,
**characterized in that**
the fishes are Cypriniformes fishes.

6. Method according to Claim 1,
**characterized in that**
the fishes are Perciformes fishes.

7. Method according to Claim 1,
**characterized in that**
the fishes are Salmoniformes fishes.

8. Method according to Claim 5,
**characterized in that**
the Cypriniformes fishes originate from the species common carp (Cyprinus carpio), goldfish (Carassius auratus), roach (Rutilus rutilus), zebrafish (Danio rerio) or fathead minnow (Pimephales promelas).

9. Method according to Claim 6,
**characterized in that**
the Perciformes fishes originate from the species European perch (Perca fluviatilis), Nile tilapia (Oreochromis niloticus), three-spined stickleback (Gasterosteus aculeatus), Japanese rice fish (Oryzias latipes) or Australian rainbow fishes (Melanotaenia species).

10. Method according to Claim 7,
**characterized in that**
the Salmoniformes fishes originate from the species brown trout (Salmo trutta forma fario), rainbow trout (Oncorhynchus mykiss) or Arctic char (Salvelinus alpinus).

11. Method according to any of the preceding claims,
**characterized in that**
a micro-reaction vessel is used as reaction vessel.

## Revendications

1. Procédé de détermination de la présence de vitellogénine dans le mucus cutané de poissons en tant que biomarqueur d'une activité oestrogénique exogène sur des poissons,
**caractérisé par** les étapes de procédé suivantes :
a) le prélèvement d'un échantillon de mucus cutané sur la région corporelle dorsale d'un poisson par raclage au moyen d'un écouvillon médico-légal comprenant une position de rupture,
b) le transfert de la partie de l'écouvillon à laquelle le mucus cutané a adhéré après la rupture à la position de rupture dans un récipient de réaction approprié,
c) l'ajout d'un tampon stabilisant les protéines dans le récipient de réaction,
d) l'homogénéisation de l'échantillon de mucus cutané,
e) le prélèvement d'une aliquote de l'échantillon de mucus cutané et la détermination de la présence de vitellogénine au moyen d'un procédé de détection ELISA approprié.

2. Procédé selon la revendication 1, **caractérisé en ce qu'**une aliquote supplémentaire de l'échantillon de mucus cutané homogénéisé est prélevée en tant qu'échantillon parallèle et une détermination supplémentaire de la présence de vitellogénine par un procédé de détection ELISA est réalisée.

3. Procédé selon la revendication 1, **caractérisé en ce qu'**une aliquote supplémentaire de l'échantillon de mucus cutané homogénéisé est prélevée et une détermination des protéines totales est réalisée pour déterminer la relation entre la teneur totale en protéines et la teneur en vitellogénine de l'échantillon de mucus cutané.

4. Procédé selon l'une quelconque des revendications 1 à 3, **caractérisé en ce que** l'aliquote pour la détermination supplémentaire est transférée sur une plaque de microtitration.

5. Procédé selon la revendication 1, **caractérisé en ce que** les poissons sont des poissons cypriniformes.

6. Procédé selon la revendication 1, **caractérisé en ce que** les poissons sont des poissons perciformes.

7. Procédé selon la revendication 1, **caractérisé en ce que** les poissons sont des poissons salmoniformes.

8. Procédé selon la revendication 5, **caractérisé en ce que** les poissons cypriniformes sont de l'espèce de la carpe commune (Cyprinus carpio), du cyprin doré (Carassius auratus), du gardon (Rutilus rutilus), du poisson-zèbre (Danio rerio) ou du tête de boule (Pimephales promelas).

9. Procédé selon la revendication 6, **caractérisé en ce que** les poissons perciformes sont de l'espèce de la perche commune (Perca fluviatilis), du tilapia (Oreochromis niloticus), de l'épinoche (Gasterosteus aculeatus), du médaka (Oryzias latipes) ou des poissons arc-en-ciel australiens (Melanotaenis spec.).

10. Procédé selon la revendication 7, **caractérisé en ce que** les poissons salmoniformes sont de l'espèce de la truite fario (Salmo trutta forma fario), de la truite arc-en-ciel (Oncorhynchus mykiss) ou de l'omble chevalier (Salvelinus alpinus).

11. Procédé selon l'une quelconque des revendications précédentes, **caractérisé en ce qu'**un récipient de micro-réaction est utilisé en tant que récipient de réaction.
